# DEMANDE DE BREVET EUROPEEN

(11) **EP 3 772 332 A1**
(43) Date de publication de la demande: **10.02.2021**
(21) Numéro de dépôt: 19290068.6
(22) Date de dépôt: 08.08.2019
(51) Int. Cl.: A61B 5/145, A61B 5/1455, A61B 5/1468

(54) **SYSTEME DE SURVEILLANCE CORPORELLE COMPRENANT UNE MICROAIGUILLE**

(71) Demandeur: PKvitality, 75001 Paris (FR)
(72) Inventeur: Pierart, Luc, 94800 Villejuif (FR)
(74) Mandataire: Regimbeau

(57) **Abrégé**

La présente invention concerne un capteur pour système de surveillance corporelle comprenant des microaiguilles de mesure d'analyte corporelle caractérisé par le fait qu'il comprend une embase support, au moins une platine support élémentaire et au moins une microaiguille répartie sur la platine support élémentaire, la platine support élémentaire étant adaptée pour être montée fixe sur l'embase support.

## Description

### DOMAINE DE L'INVENTION

La présente invention concerne un dispositif de mesure d'un analyte corporel. Plus précisément, elle concerne un dispositif de surveillance corporelle par une analyse de liquide corporel, typiquement interstitiel.

### ETAT DE LA TECHNIQUE

Certaines pathologies comme le diabète nécessitent une surveillance quotidienne de paramètres biochimiques du corps humain, en particulier des concentrations en certains composés (la glycémie dans l'exemple du glucose).

Pour cela, il est courant de piquer un point de la peau de sorte à faire perler une goutte de sang, et d'analyser cette goutte soit de façon réactive (par exemple avec une bandelette), soit de façon électronique (par exemple par au moins un capteur analytique), de façon à estimer le ou les paramètres cibles.

On connait aujourd'hui des systèmes évolués bien moins invasifs qui se contentent d'analyser le liquide interstitiel, c'est-à-dire le fluide qui remplit l'espace entre les capillaires sanguins et les cellules. Il a en effet une composition ionique proche de celle du plasma sanguin.

Ces systèmes évolués permettent ainsi de surveiller les paramètres biochimiques souhaités de façon transcutanée, sans nécessité de percer régulièrement la peau et de prélever.

En particulier, il a été proposé un dispositif porté au poignet appelé GlucoWatch, mettant en œuvre un phénomène appelé iontophorèse (ou ionophorèse) dans lequel un champ électrique permet « d'attirer » le liquide interstitiel à travers la peau jusqu'à un capteur sur la paroi du dispositif. Ce concept a cependant été abandonné rapidement car 6% seulement des patients supportaient la douleur d'extraction électrique. De surcroît les résultats des mesures étaient peu fiables.

Il a été proposé alternativement des sondes transcutanées prenant la forme d'un patch autocollant plaquant un « capteur-microaiguille » juste sous la peau, de sorte à mettre le capteur en communication fluidique permanente avec le liquide interstitiel, pour une surveillance continue. Certaines de ces sondes transcutanées de type patch comprennent des moyens de communication sans fil permettant de remonter les mesures sur le liquide interstitiel à un terminal mobile, pour un stockage et/ou un traitement des mesures (vérification de seuils et de variations, réalisation de statistiques, déclenchement d'alertes si nécessaire, *etc.*)*.* On citera par exemple les systèmes sugarBEAT™ ou FreeStyle Libre.

De nombreux systèmes proposés comprennent un réseau de micro-microaiguilles. Cependant, il s'avère que de nombreux rejets de réseaux de microaiguilles sont nécessaire lors de la fabrication d'un système en raison de défauts de quelques microaiguilles. Ceci entraîne une augmentation des coûts de production du système. Ce problème est résolu dans l'art antérieur en proposant des réseaux comprenant de nombreuses microaiguilles. Ainsi, les défauts de fabrication d'une microaiguille peuvent être minimisés lors d'une mesure d'un analyte.

### EXPOSE DE L'INVENTION

Le but principal de l'invention est de remédier aux problèmes ainsi posés.

La présente invention a en particulier pour but de proposer un capteur pour système de surveillance corporelle comprenant des microaiguilles de mesure d'analyte corporelle caractérisé par le fait qu'il comprend une embase support, au moins une platine support élémentaire et au moins une microaiguille répartie sur la platine support élémentaire, la platine support élémentaire étant adaptée pour être montée fixe sur l'embase support. L'invention est avantageusement complétée par les caractéristiques suivantes, prises individuellement ou en l'une quelconque de leurs combinaisons techniquement possibles :
- le capteur comprend plusieurs platines supports,
- le capteur comprend plusieurs microaiguilles réparties sur la platine support,
- chaque platine a des dimensions inférieures à 10x10mm, et préférentiellement inférieures à 3x3mm,
- chaque platine support élémentaire est montée fixe avec deux cents microaiguilles ou moins, avantageusement huit microaiguilles ou moins et préférentiellement avec quatre microaiguilles ou moins,
- chaque platine support élémentaire et l'embase support comportent des pistes électriques de connexion des microaiguilles,
- l'embase support et chaque platine support élémentaire comprennent des éléments à complément de forme destinés à assurer l'immobilisation des platines supports élémentaires sur l'embase support,
- les éléments à complément de forme comprennent des cavités formées dans l'embase support de géométrie complémentaire du contour des platines supports élémentaires,
- les éléments à complément de forme comprennent des bossages agencés sur l'embase support et adaptés à recevoir au moins une platine support,
- l'embase support est adaptée pour recevoir les platines supports élémentaires à des profondeurs différentes.

Un autre aspect de l'invention est un système de surveillance corporelle comprenant un capteur conforme à l'invention, et comprenant en outre un module configuré pour exploiter un signal électrique délivré par l'embase support et fournir une information représentative d'un analyte.

Un autre aspect de l'invention est un procédé de fabrication d'un capteur conforme à l'invention, comprenant au moins une étape de montage fixe d'une platine support, sur laquelle sont réparties plusieurs microaiguilles, sur une embase support, de sorte que chacune des microaiguilles soit reliée électriquement à l'embase support.

Le montage fixe de la platine support sur l'embase support est avantageusement mis en œuvre à une température inférieure à 50°C.

Un autre aspect de l'invention est un procédé de surveillance corporelle comprenant une étape dans laquelle on utilise un capteur conforme à l'invention.

### DESCRIPTION DES FIGURES

D'autres caractéristiques, buts et avantages de la présente invention apparaîtront à la lecture de la description détaillée qui va suivre, et au regard des dessins annexés, donnés à titre d'exemples non limitatifs et sur lesquels :
[Fig. 1] - la figure 1 est une vue en perspective d'un capteur comprenant quatre microaiguilles,
[Fig. 2] - la figure 2 est une vue en perspective selon un angle d'observation différent de la figure 1 d'un tel capteur,
[Fig. 3] - la figure 3 représente une vue face arrière d'un tel capteur,
[Fig. 4] - la figure 4 représente une vue latérale de ce capteur,
[Fig. 5] - la figure 5 représente une vue côté pointe du capteur,
[Fig. 6] - la figure 6 représente une vue latérale d'une microaiguille selon un mode de réalisation de l'invention,
[Fig. 7] - la figure 7 représente une vue schématique en perspective d'un système de surveillance corporelle incorporant un capteur conforme à la présente invention,
[Fig. 8] - la figure 8 représente une vue d'une capsule adaptée pour porter une pluralité de capteurs conforme à l'invention,
[Fig. 9] - la figure 9 représente une vue à échelle agrandie d'une partie de cette capsule,
[Fig. 10] - la figure 10 représente une vue à échelle agrandie d'une partie d'une capsule selon un mode de réalisation différent de l'invention,

### DEFINITION

On entend par « électrode » un dispositif conducteur permettant de capter les variations de potentiel électrique chez un organisme vivant. Une électrode comprend une borne comportant une extrémité de connexion et au moins une extrémité de détection par lesquelles un potentiel électrique ou un courant électrique est transmis, chaque extrémité de détection étant portée par une microaiguille. L'électrode peut ainsi comporter une extrémité de détection unique. Elle peut aussi comporter une pluralité d'extrémités de détection. Dans ce cas, on notera que l'électrode reste unique, quand bien même plusieurs extrémités de détection sont destinées à pénétrer dans le corps d'un organisme vivant.

### DESCRIPTION DETAILLEE DE L'INVENTION

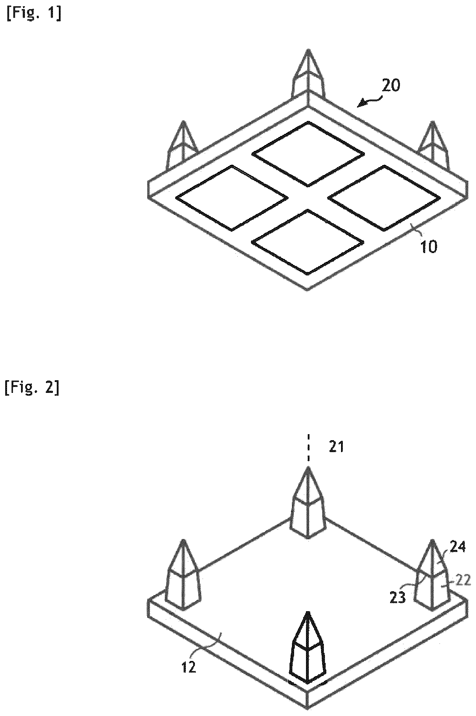

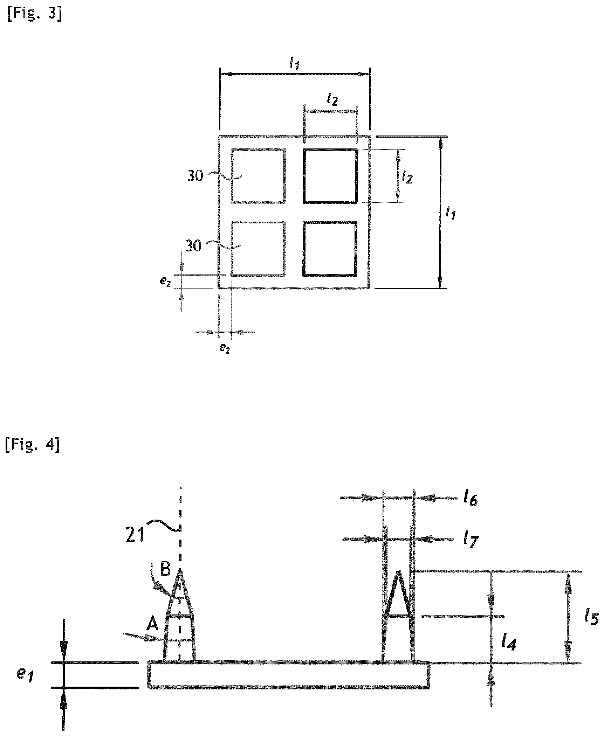

On va décrire le mode de réalisation particulier et non limitatif d'un capteur conforme à la présente invention tel qu'illustré sur les figures 1 à 6 annexées.

### Microaiguille(s)

En référence aux figures 1 à 6, un capteur comprend une platine support 10 munie de quatre microaiguilles 20. Le contour de la platine peut faire l'objet de nombreuses variantes de réalisation. Selon la représentation donnée sur les figures 1 à 5, la platine 10 a un contour carré. Les quatre microaiguilles sont situées respectivement à proximité des angles de la platine 10. Les microaiguilles 20 s'étendent perpendiculairement au plan de base de la platine 10. En d'autres termes, l'axe central 21 de chaque microaiguille 20 s'étend perpendiculairement à la surface de base de la platine 10.

En référence aux figures 1 à 3, la face de la platine 10 opposée aux microaiguilles 20 comprend quatre plages 30 électriquement conductrices, chaque plage 30 étant reliée électriquement à la partie active 25 de chaque microaiguille 20. Les plages 30 électriquement conductrices permettent une continuité électrique d'une électrode quand elles sont reliées électriquement à une embase, par exemple de travail ou d'une contre-électrode, dans la microaiguille 20, et ce potentiellement de manière séparée de sorte que chaque électrode de travail soit indépendante des autres électrodes de travail. En pratique, ces plages 30 peuvent être venues de matière avec les microaiguilles 20 ou reliées électriquement aux microaiguilles 20 par tout moyen approprié à travers ou autour de la platine 10. La platine 10 support peut être réalisée en tout matériau approprié électriquement, par exemple électriquement isolant ou conducteur. De même, les microaiguilles 20 peuvent être formées en tout matériau approprié. Elles sont propres à véhiculer un signal électrique capté par la surface active 25. A titre préférentiel, les microaiguilles 20 peuvent être formées à base de polycarbonate ou de silicium. La ou les microaiguilles 20 sont préférentiellement pleines, c'est-à-dire dénuées de cavité. Ainsi, la fabrication de microaiguilles 20 adaptées à la mesure d'analyte est facilitée, tout en permettant une mesure électrochimique d'un analyte. La microaiguille peut comprendre majoritairement du silicium. Dans le cas du silicium, la microaiguille présente une couche externe de siO₂ de protection non conductive, formée par oxydation du silicium en surface. Ainsi, la microaiguille peut ne pas comprendre de revêtement additionnel à la couche de SiO₂.

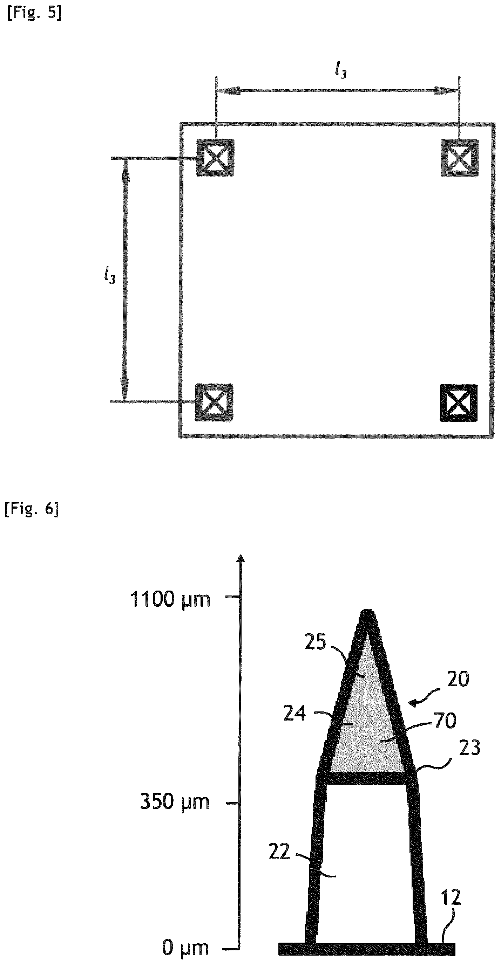

En référence à la figure 2 et à la figure 6, chaque microaiguille 20 comprend un fût de base 22 et un sommet en pointe 24. Le fût 22 peut préférentiellement s'amincir vers le sommet en pointe 24 de la microaiguille 20. Le sommet en pointe 24 présente une pente supérieure à celle du fût 22, c'est-à-dire qu'il forme un angle B avec l'axe central 21 supérieur à l'angle A formé entre fût de base 22 et l'axe central 21. La microaiguille 20 présente une cassure ou transition de pente 23 entre le fût de base 22 et le sommet en pointe. La transition de pente 23 peut être matérialisée par une arête.

Le fût de base 22 et le sommet en pointe 24 peuvent présenter une section carrée. Dans ce cas, le fût 22 de base est quadrangulaire et la pointe 24 est de type pyramidal. L'ensemble de la microaiguille 20 peut préférentiellement avoir la forme d'un obélisque.

En variante cependant, la microaiguille 20 peut présenter une section circulaire. Dans ce cas, le fût 22 de base a la forme d'un tronc de cône circulaire de révolution et la pointe 24 est formée d'une pointe conique de révolution.

De préférence, le sommet en pointe 24 s'étend exclusivement à une distance comprise entre 350 µm et 1100 µm de la base du fût de base 22 de la microaiguille, c'est-à-dire de la face 12 de la platine support 10, et préférentiellement entre 600 µm et 1000 µm de la base de la microaiguille et cette surface 12 de la platine support 10. On entend par « s'étend exclusivement à une distance comprise entre 350 µm et 1100 µm » que la partie du sommet en pointe 24 la plus proche de la base du fût de base est agencée à une distance supérieure à 350 µm de la base du fût de base 22, et que la partie la plus distante de la base du fût de base 22 est agencée à une distance inférieure à 1100 µm.

Par ailleurs, des essais ont montré que la surface de la partie active 25 de détection doit être comprise entre 0,04 et 0,9 mm². Par conséquent, lorsque la mesure est faite avec une microaiguille 20 unique, la partie active 25 de cette microaiguille 20 est comprise entre 0,04 et 0,9 mm². Lorsque la mesure est réalisée avec plusieurs microaiguilles 20, la surface de la partie active précitée, comprise entre 0,04 et 0,9 mm² s'entend de la surface totale active des microaiguilles considérées.

L'homme de l'art comprendra que la forme d'obélisque des microaiguilles 20, ou la forme d'un corps similaire circulaire de révolution mais présentant une rupture de pente entre le fût 22 de base et le sommet en pointe 24, permet de résoudre un problème posé par les microaiguilles 20 connues de l'état de l'art, à savoir minimiser le diamètre de pénétration dans la peau tout en maximisant la surface de la partie active 25 présente dans la partie de la peau comprise entre l'épiderme et les nerfs.

Les microaiguilles 20 conformes à la présente invention peuvent être réalisées à l'aide de tout procédé de microfabrication approprié.

La partie active 25 comprend une face électriquement conductrice, préférentiellement recouverte d'un revêtement faisant l'objet de diverses variantes selon le type de mesure recherché et le type d'analyte à mesurer. Pour une mesure de glycémie, la partie active 25 est pourvue d'un revêtement propre à mettre en oeuvre une réaction enzymatique avec le glucose. La partie active 25 peut également ne pas comprendre de revêtement spécifique à un analyte prédéterminé, par exemple dans le cas de la partie active 25 d'une contre-électrode ou d'une électrode de référence.

Par ailleurs, selon le mode de réalisation particulier représenté sur les figures 1 à 5, et à titre d'exemple non limitatif :
- la hauteur *l₄* du fût de base 22 est de l'ordre de 380 µm,
- la hauteur totale *l₅* de chaque microaiguille 20 est de l'ordre de 750 µm,
- la largeur à la base *l₆* de chaque microaiguille 20 est de l'ordre de 0,25mm,
- la largeur *l*₇ de la microaiguille au niveau de la transition de pente 23 est de l'ordre de 0,2mm,
- l'angle A de convergence du fût de base 22 par rapport à l'axe central 21 est de l'ordre de 7°,
- l'angle B du sommet en pointe 24 par rapport à l'axe central 21 est de l'ordre 30°.

### Réseau de microaiguilles

La ou les microaiguilles 20 conformes à l'invention permettent de réduire le nombre de microaiguilles 20 d'une électrode de travail 70. Préférentiellement, une électrode de travail comprend entre une et sept, notamment entre une et cinq et préférentiellement entre une et trois parties actives 25 recouvrant chacune au moins une partie de la surface du sommet en pointe 24 d'une microaiguille 20 différente. Les systèmes connus de l'état de l'art ne permettent pas d'utiliser aussi peu de microaiguilles.

L'invention permettant de réduire drastiquement le nombre de microaiguilles 20 nécessaires à la mesure par rapport aux systèmes connus de l'état de l'art, il est possible, pour une surface de capteur donnée, de minimiser la densité de microaiguilles. Chaque paire de microaiguilles 20 adjacentes est préférentiellement séparée d'une distance entre les pointes des sommets en pointe 24 d'au moins 1mm et préférentiellement d'au moins 1,5 mm, voir le cas échéant d'au moins 1,8mm. Ceci a pour effet d'éviter une déformation homogène de la peau lors de la mise en contact d'un réseau de microaiguilles 20 avec la peau, connue dans d'autres domaines techniques sous le nom d'effet fakir, et au contraire de favoriser une déformation de la peau localisée autour de chacune des microaiguilles. Ainsi, la douleur entraînée par la pénétration d'aiguilles dans la peau peut être significativement diminuée, voire supprimée et la pénétration se fait très naturellement, les aiguilles étant de fait devenues indépendantes mécaniquement.

Par ailleurs, selon le mode de réalisation particulier représenté sur les figures 1 à 5, et à titre d'exemple non limitatif, l'entraxe *l₃* entre chaque paire de microaiguilles 20 est de l'ordre de 1,5 mm.

### Electrodes

Le capteur est préférentiellement adapté pour mesurer la présence ou la concentration d'un analyte par électrochimie. Un capteur peut comprendre une électrode de travail 70, adaptée pour évaluer la présence d'un analyte dans le corps d'un utilisateur. L'électrode de travail 70 comprend au moins une première extrémité reliée électriquement à un module configuré pour exploiter le signal électrique de l'électrode de travail 70, et au moins une deuxième extrémité formée par la partie active 25. Elle peut également comprendre une pluralité de deuxièmes extrémités. La partie active 25 de la microaiguille 20 recouvre au moins une partie de la surface du sommet en pointe 24 et préférentiellement la totalité de la surface du sommet en pointe 24. A cet effet, la partie active 25, au niveau du sommet en pointe 24, est revêtue de tout revêtement approprié pour la mesure souhaitée, typiquement un revêtement adapté à détecter la glycémie par électrochimie.

Le capteur peut comprendre une contre-électrode. La contre-électrode peut comprendre une première extrémité destinée à être reliée électriquement à un module configuré pour exploiter un signal électrique, et au moins une autre extrémité permettant d'exploiter un signal électrique dans le corps de l'utilisateur. L'autre extrémité de la contre-électrode peut recouvrir une microaiguille de contre électrode, par exemple une microaiguille conforme à l'invention. Cependant, la contre-électrode n'a pas les mêmes prérequis de surface active que l'électrode de travail. Ainsi, le sommet en pointe de la contre-électrode peut s'étendre exclusivement à une distance comprise entre 100 µm et 1100 µm du fût de base de la microaiguille. En variante, l'autre extrémité de la contre-électrode peut recouvrir toute la surface de la microaiguille de contre-électrode.

Dans le cas d'un capteur conforme à un mode de réalisation de l'invention comprenant plusieurs électrodes de travail, chaque électrode de travail peut être adaptée à détecter le même analyte qu'une autre électrode de travail, ou être adaptée à détecter un analyte différent d'une autre électrode de travail.

Dans le cas où plusieurs électrodes de travail sont adaptées à détecter le même analyte, chaque électrode de travail peut comprendre une partie active 25 comprenant le même type de revêtement. Ainsi, il peut être possible de mettre en oeuvre des mesures indépendantes pour le même analyte, et ainsi d'obtenir une meilleure précision de mesure de l'analyte. Chaque électrode de travail peut également comprendre des parties actives 25 différentes, comprenant des revêtements différents, mais adaptés à détecter le même analyte. La concentration de l'analyte peut ainsi être détectée avec plus de précision qu'en utilisant un seul revêtement pour la partie active 25.

Chaque électrode peut également être adaptée à détecter des analytes différents. Ainsi, il est possible de surveiller plusieurs pathologies avec le même système de surveillance.

### Platine support

Comme vu précédemment, la ou les microaiguilles 20 peuvent être agencées sur une platine support 10. En référence aux figures 1 à 4, l'épaisseur e₁ de la platine support est avantageusement comprise entre 0,1mm et 1 mm, et préférentiellement de l'ordre de 0,2 mm. Les dimensions des microaiguilles 20 peuvent faire l'objet de nombreuses variantes de réalisation. Il en est de même pour la platine support 10. A titre d'exemple non limitatif en référence au mode de réalisation particulier représenté sur les figures 1 à 5, la platine support 10 présente des côtés ayant une largeur l₁ inférieure à 10mm, avantageusement inférieure à 3mm, par exemple de l'ordre de 2,3mm. Les plages électriquement conductrices 30 sont des plages par exemple carrées ayant un côté *l₂* de l'ordre de 0,8mm. Ces plages 30 peuvent être situées à une distance e₂ de l'ordre de 0,2 mm des bords de la platine support.

Les platines supports 10 peuvent elles-mêmes faire l'objet de différentes variantes de réalisation. Certaines platines supports 10 peuvent être adaptée à supporter par exemple quatre microaiguilles tandis que d'autres platines supports 10 peuvent être adaptées à supporter seulement deux microaiguilles 20.

La ou les platines supports 10 sont adaptées à être montées sur une embase support 80. L'embase support 80 peut être par exemple le boîtier 40, une capsule 50, ou une structure permettant de recevoir le boîtier 40, par exemple liée aux moyens d'attache corporelle.

Chaque platine support 10 peut être montée fixe avec huit microaiguilles 20 ou moins, et préférentiellement avec quatre microaiguilles 20 ou moins. Ainsi, pour un taux de défaillance de fabrication d'une microaiguille 20 dans un réseau de microaiguille 20 donné, la fréquence de fabrication d'un réseau de microaiguille 20 dans lequel toutes les microaiguilles 20 sont valides augmente par rapport aux réseaux de microaiguilles comprenant par exemple plus de 50 ou plus de 100 microaiguilles.

Chaque platine support 10 et l'embase 80 comprennent préférentiellement des pistes électriques de connexion des microaiguilles 20. Ainsi, une électrode, par exemple une électrode de travail ou une contre électrode, peut traverser la platine support 10, de sorte à avoir une première extrémité dans l'embase support et au moins une autre extrémité sur une microaiguille 20. Il est également possible d'utiliser une colle électriquement conductrice de sorte à monter fixe la platine support 10 sur l'embase support 80 et à permettre une connexion électrique entre l'embase support 80 et la platine support 10, et par extension la microaiguille 20.

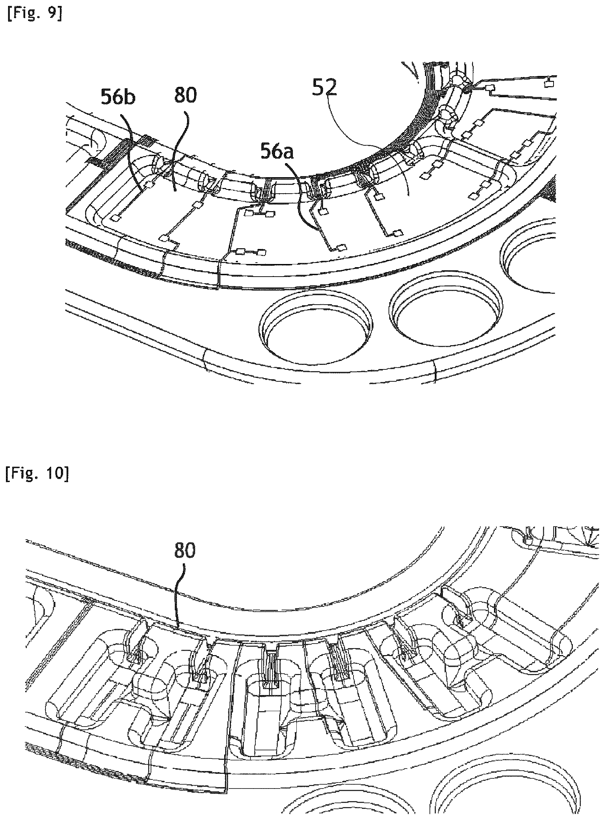

L'embase 80 et la ou les platine support 10 comprennent préférentiellement des éléments à complément de forme destinés à assurer l'immobilisation des platines supports 10 élémentaires sur l'embase support 80. En référence à la figure 10, un élément à complément de forme peut être préférentiellement une cavité formée dans l'embase support 80, de géométrie complémentaire au contour d'une ou de plusieurs platines support 10. En variante ou en complément, l'élément à complément de forme peut être préférentiellement des bossages agencés sur l'embase de support 80 et adaptés à recevoir au moins une platine support 10, par exemple de manière à l'entourer et/ou à la maintenir. Ainsi, lors du montage des platines support 10 sur l'embase 80, la platine support 10 et les microaiguilles peuvent être maintenues dans une position contrôlée, jusqu'à la fixation de la ou des platines support 10 sur l'embase 80. De plus, chacune des platines support 10 peut ainsi être montée fixe dans une position parfaitement contrôlée.

L'embase support 80 peut être préférentiellement adaptée pour recevoir les platines supports 10 élémentaires à des profondeurs différentes. Ainsi, il est possible de fabriquer un système comprenant des microaiguilles 20 ayant des hauteurs différentes, les microaiguilles 20 étant fabriquées par un procédé dans lequel toutes les microaiguilles 20 obtenues par le procédé de fabrication ont la même taille.

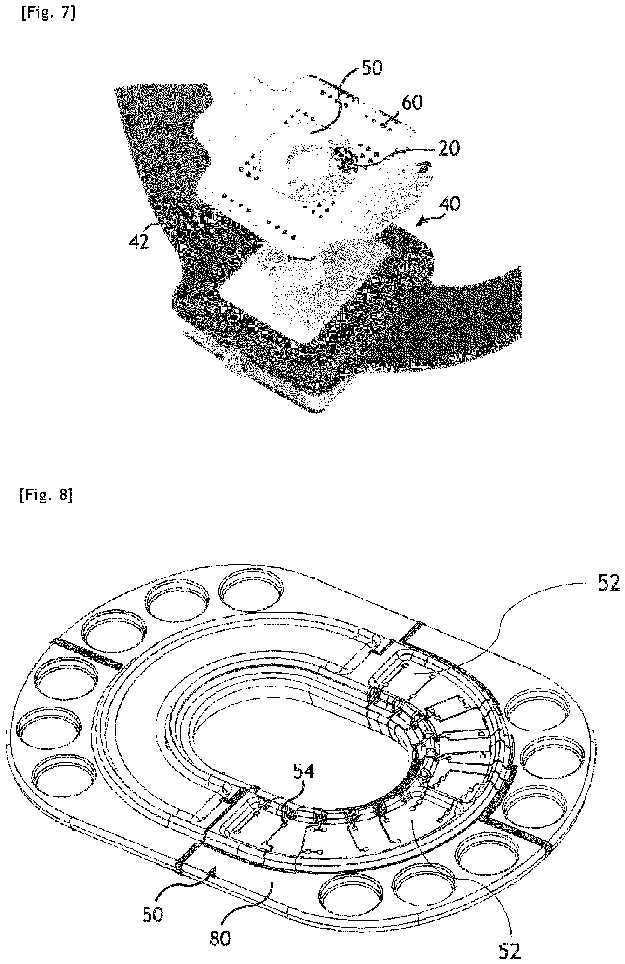

En référence aux figures 8 et 9, l'embase support 80 peut comprendre des pistes électriques. Les éléments à complément de forme de l'embase 80 et les pistes électriques de l'embase 80 peuvent être agencées de sorte qu'une platine support 10 présente également une ou plusieurs pistes électriques en regard de la piste électrique de l'embase 80. Ainsi, une électrode, par exemple une électrode de travail ou une contre-électrode peut être formée à cheval sur l'embase et sur la platine support 10.

Un aspect de l'invention est un procédé de fabrication d'un système de surveillance corporelle conforme à l'invention, dans lequel on monte fixe sur l'embase support 80 la ou les platines support 10 sur laquelle ou lesquelles sont réparties les microaiguilles 20, de sorte que chacune des microaiguilles 20 soit reliée électriquement à l'embase support 80, séparément ou pas. Ainsi, il est possible de contrôler, à partir de l'embase support 80, le potentiel des parties d'électrode de la platine support 10 et de chaque microaiguille 20.

Chaque microaiguille 20 peut présenter une partie active 25 comprenant un revêtement comprenant un composé actif biologique qui peut être dénaturé par des températures trop élevées. L'étape de montage fixe de la platine support 10 est préférentiellement mise en œuvre à une température inférieure à 80°C, notamment inférieure à 50°C. Ainsi, on évite la dénaturation des composés actifs des microaiguille 20. Cette étape de montage fixe peut être mise en oeuvre par collage par irradiation aux rayons ultra-violets entre la platine support 10 et l'embase support 80, à l'aide d'une colle électriquement conductrice.

### Système de mesure

La présente invention peut permettre de mesurer de manière indépendante le niveau de glycémie à l'aide d'une pluralité d'électrode de travail 70. Ceci présente un avantage indéniable par rapport à l'état de l'art selon lequel une telle mesure indépendante à l'aide d'une électrode de travail comprenant une microaiguille unique n'était pas envisageable car le signal de mesure était trop bruité en utilisant une seule microaiguille. Ainsi, le capteur comprend préférentiellement plusieurs électrodes de travail 70, le système de mesure étant adapté à mesurer individuellement le potentiel électrique de chacune des électrodes de travail 70.

La mesure du potentiel de chacune des électrodes de travail 70 peut être multiplexée. Dans le cadre de la présente invention, il est proposé avantageusement des moyens permettant de rejeter les valeurs de mesure minimales ou maximales du potentiel associé à un ensemble de mesures, ce qui était impossible en utilisant les capteurs de l'art antérieur.

Le capteur conforme à la présente invention peut être mis en œuvre dans différents types de système de surveillance corporelle.

De préférence, le capteur conforme à l'invention est mis en œuvre dans un système de surveillance du type illustré sur les figures 7 à 10 annexées.

Un tel système comprend un boîtier 40 en forme de boîtier de montre comportant un bracelet 42 adapté pour entourer le poignet d'un individu. Le boîtier 40 loge un module configuré pour exploiter le signal électrique délivré par chaque microaiguille 20 et fournir une information représentative d'une grandeur physique du fluide, typiquement d'un taux de glycémie.

Comme on l'a évoqué précédemment, le système de surveillance corporelle utilisé préférentiellement selon l'invention comprend une capsule 50 comprenant au moins un capteur du type précité, et de préférence une pluralité de capteurs comme on le décrira plus en détail par la suite.

Le système de surveillance corporelle conforme à l'invention comprend par ailleurs un patch 60 auquel est liée la capsule 50, le patch 60 étant lui-même pourvu d'un adhésif permettant de faire adhérer l'ensemble patch et capsule 50 sur la peau d'un individu.

Comme on l'a représenté sur la figure 8 et en partie sur la figure 9, la capsule 50 a de préférence la forme générale d'un anneau comportant une pluralité de logements en creux 52 adaptés pour recevoir chacun respectivement la platine support 10 d'un capteur précité.

En référence aux figures 7 et 8, la capsule 50 peut comprendre des plages électriquement conductrices 54 destinées à être placées en regard des plages électriquement conductrices 30 prévues sur la platine support 10, pour assurer une liaison électrique entre les microaiguilles 20 et le module prévu dans le boîtier 40 pour exploiter le signal électrique ainsi prélevé. Les plages 54 sont elles-mêmes interconnectées avec le module précité par des pistes électriquement conductrices 56a.

Comme on le voit à l'examen de la figure 9, certaines des plages 54 peuvent être reliées individuellement au module de traitement précité par des pistes 56a respectives tandis que d'autres plages 54 peuvent être reliées au module de traitement par des pistes communes 56b.

### Mise en œuvre d'une mesure d'analyte corporel

Comme on l'a indiqué précédemment, la présente invention concerne également un procédé de surveillance corporelle à l'aide d'un capteur comportant une microaiguille du type précité.

Le procédé de surveillance comprend une étape de mesure d'analyte corporel à l'aide d'une microaiguille 20 conforme à un mode de réalisation de l'invention. De par les caractéristiques de la microaiguille 20, le capteur peut comprendre une pluralité d'électrodes de travail. La mesure peut par exemple être mise en oeuvre en polarisant la ou les électrodes de travail et la ou les contre-électrodes à un potentiel électrique adapté pour entraîner une réaction d'oxydo-réduction impliquant l'analyte à mesurer.

L'étape de mesure peut être préférentiellement mise en œuvre au moins à l'aide de deux électrodes de travail différentes. L'étape de mesure peut être par exemple mise en œuvre indépendamment, successivement sur chacune des électrodes de travail 70, ou en parallèle sur chacune des électrodes de travail 70. Ainsi, la concentration de l'électrolyte peut être analysée de manière plus précise qu'avec un système comprenant par exemple une seule électrode de travail présentant plusieurs extrémités sous forme de microaiguilles.

Un autre aspect de l'invention est un procédé de mesure d'analyte corporel comprenant une étape de pénétration des microaiguilles d'un capteur conforme à l'invention dans la peau d'un utilisateur. Ainsi, les aiguilles du capteur peuvent être introduites dans la peau sans applicateur, de par l'espacement des microaiguilles 20. Une force basse en comparaison de la force procurée par un applicateur peut être utilisée pour la pénétration des microaiguilles. Préférentiellement, une force inférieure à 50 newtons, et préférentiellement inférieure à 30 newtons, peut être utilisées pour la pénétration des microaiguilles 20. Ainsi, la pénétration des microaiguilles peut être mise en œuvre avec la main, ou préférentiellement avec des moyens d'attache mécanique du système, par exemple un bracelet.

## Revendications

1. Capteur pour système de surveillance corporelle comprenant des microaiguilles (20) de mesure d'analyte corporelle **caractérisé par le fait qu'**il comprend une embase support (80), au moins une platine support (10) élémentaire et au moins une microaiguille (20) répartie sur la platine support (10) élémentaire, la platine support (10) élémentaire étant adaptée pour être montée fixe sur l'embase support (80).

2. Capteur selon la revendication 1, **caractérisé par le fait que** la platine support (10) élémentaire est montée fixe avec deux cents microaiguilles (20) ou moins, avantageusement huit microaiguilles au moins et préférentiellement quatre microaiguilles (20) ou moins.

3. Capteur selon la revendication 1 ou 2, **caractérisé par le fait que** la platine support (10) élémentaire et l'embase support (80) comportent des pistes électriques de connexion des microaiguilles (20).

4. Capteur selon l'une des revendications 1 à 3, **caractérisé par le fait que** la platine support (10) élémentaire est inférieure à 10x10mm, et préférentiellement inférieure à 3x3mm.

5. Capteur selon l'une des revendications 1 à 4, **caractérisé par le fait que** l'embase support (80) et chaque platine support (10) élémentaire comprennent des éléments à complément de forme destinés à assurer l'immobilisation des platines supports (10) élémentaires sur l'embase support.

6. Capteur selon la revendication 5, **caractérisé par le fait que** les éléments à complément de forme comprennent des cavités formées dans l'embase support (80) de géométrie complémentaire du contour des platines supports (10) élémentaires.

7. Capteur selon la revendication 5 ou 6, **caractérisé par le fait que** les éléments à complément de forme comprennent des bossages agencés sur l'embase support (80) et adaptés à recevoir au moins une platine support (10).

8. Capteur selon l'une des revendications 1 à 7, **caractérisé par le fait que** l'embase support (80) est adaptée pour recevoir les platines supports (10) élémentaires à des profondeurs différentes.

9. Système de surveillance corporelle **caractérisé par le fait qu'**il comprend un capteur conforme à l'une des revendications 1 à 8 et qu'il comprend en outre un module configuré pour exploiter un signal électrique délivré par l'embase support (80) et fournir une information représentative d'un analyte.

10. Procédé de fabrication d'un capteur conforme à l'une des revendications 1 à 8, comprenant au moins une étape de montage fixe d'une platine support (10) sur laquelle sont réparties plusieurs microaiguilles (20), sur une embase support (80), de sorte que chacune des microaiguilles (20) soit reliée électriquement à l'embase support (80).

11. Procédé de fabrication conforme à la revendication 10, dans lequel le montage fixe de la platine support (10) sur l'embase support (80) est mis en œuvre à une température inférieure à 50°C.
